# EUROPÄISCHE PATENTSCHRIFT

(11) **EP 1 243 259 B1**
(45) Veröffentlichungstag und Bekanntmachung des Hinweises auf die Patenterteilung: **28.07.2004**
(21) Anmeldenummer: 02004978.9
(22) Anmeldetag: 06.03.2002
(51) Int. Cl.: A61K 9/70, A61K 47/32, A61K 47/10, A61K 31/7036, A61L 31/10, A61L 29/08, A61L 31/16, A61L 29/16

(54) **Antibiotikum-/Antibiotika-Polymer-Kombination mit retardierender Wirkstofffreisetzung**
Sustained release preparation comprising one or more antibiotics and polymers
Préparation à libération prolongée contenant un ou plusieurs antibiotiques et des polymères

(30) Priorität: 22.03.2001 DE 10114247
(43) Veröffentlichungstag der Anmeldung: 25.09.2002
(73) Patentinhaber: Heraeus Kulzer GmbH & Co.KG, 63450 Hanau (DE)
(72) Erfinder: Vogt, Sebastian F., Dr., 07749 Jena (DE); Schnabelrauch, Matthias, Dr., 07745 Jena (DE); Kühn, Klaus-Dieter, Dr., 35041 Marburg (DE)
(74) Vertreter: Kühn, Hans-Christian

(56) Entgegenhaltungen:
- EP-A- 0 020 905
- EP-A- 0 086 997
- WO-A-92/11845
- US-A- 5 160 737

## Beschreibung

Die vorliegende Erfindung betrifft eine Antibiotikum-/Antibiotikum-Polymer-Kombination, die unter physiologischen Bedingungen eine kontinuierliche Freisetzung von Antibiotika über einen Zeitraum von mehreren Tagen gewährleistet und in der Human- und Veterinärmedizin eingesetzt werden kann.

In der Human- und Veterinärmedizin werden Medizinprodukte aus Kunststoffen in Form von Drainagen, Kathetern, Abdeckfolien und Netzen als temporäre oder dauerhafte Implantate zur Sekretabsaugung, Spülung, Abdeckung und Fixierung eingesetzt. Problematisch ist hierbei, daß Mikroorganismen insbesondere bei Drainagen und Kathetern entlang dieser Kunststoffschläuche in den Organismus einwandern können und dadurch lokale Infektionen verursachen, die sich unbehandelt weiter im Organismus ausbreiten können. Ähnliche Probleme treten beim Einsatz von Fixateuren externe auf. Dabei können Mikroorganismen in gleicher Weise entlang der Verstiftungen in den Organismus eindringen. Auch bei Dentalimplantaten sind Infektionsprobleme an den Implantatoberflächen bekannt. Hieraus ergibt sich die Notwendigkeit, bei der medizinischen Anwendung dieser Implantate eine Infektionsprophylaxe bzw. eine Infektionsbekämpfung vorzunehmen. Diese Infektionsunterdrückung kann grundsätzlich systemisch oder lokal mit geeigneten Antibiotika erfolgen. Die systemische Anwendung von Antibiotika ist mit einer Reihe von Problemen behaftet. Um antimikrobiell-wirksame Antibiotika-Konzentrationen systemisch erreichen zu können, sind relativ hohe Antibiotika-Dosen erforderlich. Dadurch kann es insbesondere bei den Antibiotika des Aminoglykosid-Typs und bei den Antibiotika des Tetracyclin-Typs zu unerwünschten Schädigungen auf Grund ihrer Nephrotoxizität bzw. Ototoxizität kommen. Daher ist eine Infektionsunterdrückung durch lokale Anwendung von Antibiotika sinnvoller, weil hierbei wirksame lokale Antibiotika-Konzentrationen erreicht werden können, unter Vermeidung von hohen systemischen Antibiotika-Konzentrationen.

Die Herstellung und Verwendung von antibiotischen Polymer-Kompositen ist seit Jahren Gegenstand intensiver Forschungen, die zu einer Reihe von Patenten führten. So legten Shepherd und Gould eine Beschichtung von Kathetern mit hydrophilen Polymethacrylaten und Polyacrylaten offen, in die ein nicht näher spezifiziertes Antibiotikum zur Behandlung von Infektionen eingebracht ist (T. H. Shepherd, F. E. Gould: Catheter. 03.03.1971 **US 3,566,874**). Ebenfalls von Shepherd und Gould stammt ein in den 1970er Jahren beschriebenes Retardsystem auf Basis von hydrophilen Hydroxyalkylacrylaten und Hydroxymethacrylaten, die zu antibiotisch ausgrüsteten Formkörpern polymerisiert werden (T. H. Shepherd, F. E. Gould: Dry hydrophilic acrylate or methacrylate polymer prolonged release drug implants. 31.12.1974 **US 3,857,932**). Klemm beschrieb aus Polymethacrylat und Polyacrylat aufgebaute Kunststoffpartikel zur Behandlung von Osteomyelitis (K.Klemm: surgical synthetic-resin material and method of treating osteomyelitis. 13.05.1975 **US 3,882,858**). Diese Kunststoffpartikel waren mit Gentamicin oder einem anderen Antibiotikum impregniert. Von Gross et al. stammt ein fortgeschrittener Vorschlag zur Herstellung von Knochenzement, der Gentamicin enthält (A. Gross, R. Schaefer, S. Reiss: Bone cement compositions containing gentamycin. 22.11.1977 **US 4,059,684**). Hierbei werden als Hilfsstoffe in Wasser leicht lösliche Salze wie Natriumchlorid, Kaliumchlorid, Natriumbromid und Kaliumbromid zu einem Gemisch bestehend aus gepulverten Kopolymeren von Methylmethacrylat und Methylacrylat, Methylmethacrylat, Gentamicinhydrochlorid und/oder Gentamicinsulfat gegeben. Diese Mischung wurde durch Peroxide polymerisiert. Die in Wasser leicht löslichen Salze lösen sich nach Einbringung des Knochenzementes in physiologisches Milieu und hinterlassen Hohlräume. Von Batich et al. wurde ein neues Freisetzungssystem auf Grundlage von Kopolymeren beschrieben, das unter Verwendung von schwach sauren Monomeren synthetisiert wurde, und das ab einem pH-Wert von 8,5 quillt und dadurch eine Wirkstofffreisetzung von eingeschlossenen pharmazeutischen Wirkstoffen ermöglichen soll (C D. Batich, M. S. Cohen, K. Forster: Compositions and devices für controlled release of active ingredients. 10.10.1996 **US 5,554,147**).

Der Gegenstand einer Reihe weiterer Arbeiten war die antimikrobielle Beschichtung von Medizinprodukten mit antibiotischen Polymersystemen. So entwickelten Conway et al. eine Polymermatrix aus Silicon, in die in Wasser lösliche Wirkstoffe auf Nitrofuran-Basis fein verteilt eingeschlossen wurden (A. J. Conway, P. J. Conway, R.D. Fryar Jr.: Sustained release bactericidal cannula. 16.11.1993 **US 5,261,896**). Die Verwendung eines Matrix-bildenden Polymers aus der Gruppe der Polyurethane, der Silicone und der bioabbaubaren Polymere, in denen ein Gemisch aus einem Silbersalz und Chlorhexidin suspendiert ist, wurde zur Herstellung von Infektions-resistenten Medizinprodukten offengelegt ( C. L. Fox Jr., S. M. Modak, L. A. Sampath: Infection-resistant compositions, medical devices and surfaces and methods for preparing and using same. 28.05.19991 **US 5,019,096**). Von Solomon, Byron und Parke wurden ähnliche antiinfektive Systeme auf Basis von Polyurethan und darin dispergiertem Chlorhexidin vorgeschlagen (D. D. Solomon, M P. Byron: Anti-infective and antithrombogenic medical articles and method for their preparation. 19.09.1995 **US 5,451,424**; D. D. Soloman, M. P. Parke: Anti-infective and antithrombogenic medical articles and method for their preparation. 13.01.1998 **US 5,707,366**; D. D. Soloman, M. P. Parke: Anti-infective and antithrombogenic medical articles and method for their preparation. 13.01.1998 **US 5,165,952**). Diese Systeme konnten aus der Schmelze durch Extrusion zu Formkörpern verarbeitet werden. Eine antibiotische Komposition, die aus oligodynamisch wirkenden Metallen und Polymeren zusammengesetzt ist, wurde ebenfalls offengelegt (D. Laurin, J. Stupar: Antimicrobial compositions. 29.07.1984 **US 4,603,152**). Als Polymere werden Acrylnitril-Butadien-Styren-Kopolymere, Polyvinylchlorid, Polyester, Polyurethane, Styren-Block-Kopolymere und Gummi vorgeschlagen in denen oligodynamisch wirksame Metalle zur Infektionsunterdrückung eingebracht sind. Auch Elastomere konnten antibiotisch ausgerüstet werden. So erzeugte Allen Elastomer-Wirkstoffkombinationen durch Hinzumischung und Einarbeitung von Wirkstoffen in Gummi-Masterbatchs (D. L. Allen: Elastomeric composition containing therapeutic agents and articles manufactured therefrom. 28.05.1991 **US 5,019,378**). Die Masterbatchs setzten sich aus Gummi, Glimmer und Titandioxid zusammen. Eine antibiotische Beschichtung bestehend aus einem Gemisch von Rifampin und Minocyclin, die in einem Polymer dispergiert wurden, schlagen Raad und Darouiche vor (I. I. Raad, R. O. Darouiche: Antibacterial coated medical implants. 08.06.1993 **US 5,217,493**). Das Polymermaterial ist hierbei nicht näher charakterisiert. De Leon et al. legen eine Methode zur antibiotischen Beschichtung von Implantaten offen, bei denen die zu beschichtende Oberfläche zuerst mit Siliconöl beschichtet wird ( J. De Leon, T. H. Ferguson, D. S. Skinner Jr.: Method of making antimicrobial coated implants. 28.03.1990 **US 4,952,419**). Auf die Siliconöl-Schicht bringt man in einem zweiten Schritt den gepulverten Wirkstoff auf. Oxytetracyclin wurde hierbei als Wirkstoff verwendet. Eine ähnliche Beschichtung auf Basis von Silicon-Öl und Poly(methacrylsäureester) wurden von Takigawa beschrieben, die ausgehend von einer Lösung von Silicon-ÖI und Poly(methacrylsäureester) in Terpentin-Öl, N-Decane, Tetrachlormethan, Butan-2-on, 1,4-Dioxan, Ethoxyethanol und Toluen hergestellt wurde ( B. Takigawa: Coating solution containing silicone oil and polymethacrylate. 24.1998 **US 5,721,301**). Mustacich et al. beschreiben eine antimikrobielle Polymer-Kombination, bei der Fettsäuren und Fettsäuresalze als biozide Reagenzien in medizinisch anwendbare Polymere eingebracht werden (R.V. Mustacich, D. S. Lucas, R. L. Stone: Antimicrobial polymer compositions. 30.10.1984 **US 4,479,795**). Eine interessante Beschichtungs-Komposition wurde von Whitbourne und Mangan offengelegt, bei der quartäre Ammoniumverbindungen als antimikrobielles Reagens in ein Wasser unlösliches Polymer, zum Beispiel in Celluloseester, eingearbeitet sind (R. J. Whitbourne, M. A. Mangan: Coating compositions comprising pharmaceutical agents. 11.06.1996 **US 5,525,348**). Von Friedmann et al. wurden eine Reihe von Patenten bekannt, die sich mit der Herstellung von Dentallacken befassen (M. Friedmann, D. Steinerg, A.Soskolne: Sustained-release pharmaceutical compositions. 11.06.1991 **US 5,023,082**; M. Friedman, A. Sintov: Liquid polymer composition, and method of use. 03.11.1992 **US 5,160,737**; M. Friedman, A. Sintov: Dental varnish composition, and method of use. 19.07.1994 **US 5,330,746**; M. Friedman, A. Sintov: Dental varnish composition and method of use. 15.07.1997 **US 5,648,399**; M. Friedman, A. Sintov: Dental varnish composition, and method of use.17.06.1997 **US 5,639,795**). Diese Patente sind inhaltlich nahezu gleich und enthalten als wesentliche antimikrobielle Substanzen quarternäre Ammoniumsalze. Es werden in den Patenten Lacke und Polymerlösungen zu deren Herstellung beschrieben, die im wesentlichen aus folgenden Komponenten bestehen: einem aus Methacrylsäure und Methacrylsäureestern aufgebauten Kopolymeren mit freien Carbonsäuregruppen, einem aus Methacrylsäure und Methacrylsäuremethylester aufgebauten Kopolymeren mit freien Carbonsäuregruppen, einem aus Dimethylaminoethylacrylat und Ethylmethacrylat aufgebauten Kopolymeren und einem aus Methylacrylat und Chlortrimethylammoniumethylmethacrylat gebildeten Kopolymer. Bei **US 5,648,399** ist interessant, daß ein die Wirkstofffreisetzung beeinflussendes Reagens aus der Gruppe der quervernetzenden Reagenzien, der Polysaccharide, der Lipide, der Polyhydroxyverbindungen, der Polycarbonsäuren, divalente Kationen, Zitronensäure, Natriumzitrat, Natriumdocusat, Proteine, Polyoxyethylensorbitanmonooleat und Aminosäuren der Polymer-Kombination zugesetzt wird.

Von Bayston und Grove stammt ein interessanter Vorschlag zur Herstellung von antimikrobiellen Medizinprodukten (R. Bayston, N. J. Grove: Antimicrobial device and method. 17.04.1990 **US 4,917,686**). Hierbei werden antibiotische Substanzen in einem geeigneten organischen Lösungsmittel gelöst. Diese Lösung läßt man auf die zu modifizierenden Polymeroberflächen einwirken. Durch das Lösungsmittel quillt das Polymer an und der Wirkstoff kann in die Oberfläche eindringen. Darouiche und Raad schlagen eine prinzipiell gleiche Methode zur antimikrobiellen Impregnierung von Kathetern und anderen medizinischen Implantaten vor, bei denen ebenfalls ein antimikrobieller Wirkstoff in einem organischen Lösungsmittel gelöst wird (R. Darouche, I. Raad: Antimicobial impregnated catheters and other medical implants and method for impregnating catheters and other medical implants with an antimicrobial agent. 29.04.1997 **US 5,624,704**). Diese Lösung läßt man auf die zu behandelnde Oberfläche einwirken, wobei der Wirkstoff in das Material eindringt und sich dort ablagert.

Eine Alternative zu den bisher beschriebenen Systemen stellt eine von Lee beschriebene Methode zur Oberflächenbeschichtung mit kationischen Antibiotika dar (C. C. Lee: Coating medical devices with cationic antibiotics. 23.01.1990 **US 4,895,566**). Bei dieser Methode wird zuerst eine negativ geladene Heparinschicht auf die zu beschichtende Oberfläche aufgebracht und anschließend nach deren Anheftung läßt man an diese kationische Antibiotika anlagern. Eine ähnliche Lösung schlagen Greco et al. vor, bei der man zuerst eine Lösung von anionischen oberflächenaktiven Substanzen auf die zu beschichtende Oberfläche einwirken läßt (R. S. Greco, R. A. Harvey, S. Z. Trooskin: Drug bonded prosthesis and process for producing same. 07.11. 1989 **US 4,879,135**). Dabei adsorbieren die anionischen Moleküle auf der Oberfläche. Anschließend werden kationische Wirkstoffe, wie zum Beispiel Gentamicin, elektrostatisch angebunden. Es muß bei den beiden zuletzt zitierten Verfahren angemerkt werden, daß die Beladungsdichte mit Antibiotika pro Flächeneinheit sehr begrenzt ist und daß die Haftfestigkeit dieser Beschichtungen kritisch zu sehen ist.

Der vorliegenden Erfindung liegt die Aufgabe zugrunde, eine flexible Antibiotikum-/Antibiotika-Polymer-Kombination zu entwickeln, die unter physiologischen Bedingungen eine kontinuierliche Freisetzung von Antibiotika über einen Zeitraum von mehreren Tagen bis zu Wochen erlaubt und in der Human- und Veterinärmedizin eingesetzt werden kann. Diese Antibiotikum/Antibiotika-Polymer-Kombination soll in einfacher Weise haftfest auf die Oberflächen von medizinischen Kunststoff- und Metallimplantaten aufgebracht werden können. Hierbei ist es insbesondere wichtig, daß die Beschichtung flexibel und elastisch ist und keine toxischen Komponenten abgegeben werdem. Weiterhin soll die flexible Antibiotikum-/Antibiotikum-PolymerKombination zur Herstellung von antibiotischen Fäden, Folien und Formkörpem geeignet sein.

Der Erfindung liegt der überraschende Befund zugrunde, daß homogene Polymermischungen bestehend aus einem oder mehreren hydrophoben Polymeren aus den Gruppen der Poly(methacrylsäureester), der Poly(acrylsäureester), der Poly(methacrylsäureester-coacrylsäureester) und ein oder mehreren hydrophilen Polymeren aus der Gruppe der Polyether, in denen ein oder mehrere in Wasser gering lösliche Antibiotika aus den Gruppen der Aminoglykosid-Antibiotika, der Lincosamid-Antibiotika, der Tetracyclin-Antibiotika und Chinolon-Antibiotika suspendiert sind, stabile Komposite bilden, die im wässrigen Milieu eine Antibiotika-Freisetzung über einen Zeitraum von Tagen zeigen. Die nachfolgende Erklärung ist eine beschreibende Deutung der vermutlich ablaufenden folgenden Vorgänge. Nach Einbringung der Komposite in wässriges Milieu löst sich der hydrophile Polyether und geht in Lösung, wobei die hydrophoben, in Wasser nicht löslichen Polymere zurückbleiben. Es entstehen so mikroporöse, interkonnektierende Hohlräume in der verbleibenden hydrophoben Polymer-Matrix. Das heißt, erst unter Einwirkung von wässrigen bzw. physiologischem Milieu findet unter in situ Bedingungen die Bildung von mikroporösen, interkonnektierenden Hohlräumen statt. In dieser verbleibenden hydrophoben Polymer-Matrix sind die in Wasser geringlöslichen Antibiotika-Partikel physikalisch eingeschlossen. Durch die gebildeten Hohlräume kann das wässrige Milieu, erst nach deren Entstehung, die in Wasser gering löslichen Antibiotika erreichen. Die Antibiotika-Freisetzung beginnt somit erst während bzw. nach Auslaugung der Polyether.

Diese hydrophilen Polymere sind toxikologisch unbedenklich und einige ihrer Vertreter sind in der europäischen Pharmakopoe beschrieben. Der besondere Vorteil dieser Antibiotikum/Antibiotika-Polymer-Kombination besteht darin, daß die in der homogenen Polymermischung suspendierten Antibiotika vor der Einbringung in wässriges, physiologisches Milieu vor chemischen und mechanischen Einflüßen, wie zum Beispiel Abrasion, geschützt sind. Erst durch die in situ Bildung der mikroporösen, interkonnektierenden Hohlräume wird die Antibiotikum/Antibiotika-Polymer-Kombination für die Freisetzung der Antibiotika geöffnet. Durch die Verwendung von in Wasser gering löslichen Antibiotika werden diese nur langsam aus den interkonnektierenden Hohlräumen herausgelöst. Es zeigte sich darüber hinaus überraschend, daß durch den Anteil an hydrophilen Polyether in der homogenen Polymermischung die Freisetzungsgeschwindigkeit der Antibiotika beeinflußt werden kann.

Die Aufgabe der Erfindung wurde dadurch gelöst, daß in einer homogenen Polymermischung, die aus einem oder mehreren hydrophoben Polymeren aus den Gruppen der Poly(methacrylsäureester), der Poly(acrylsäureester) und der Poly(methacrylsäureester-coacrylsäureester) und aus einem oder mehreren hydrophilen Polymeren aus den Gruppen der Polyether besteht, ein oder mehrere in Wasser gering lösliche Antibiotika aus den Gruppen der Aminoglykosid-Antibiotika, der Lincosamid-Antibiotika, der Tetracyclin-Antibiotika, der Chinolon-Antibiotika, einem in Wasser leichtlöslichem Antibiotikum aus den Gruppen der Aminoglykosid-Antibiotika, der Lincosamid-Antibiotika, der β-Lactam-Antibiotika und der Tetracyclin-Antibiotika, und ggf. ein oder mehrere organische Hilfsstoffe, suspendiert sind und diese Suspension ein Komposit bildet.

Die nachfolgenden Ausführungsformen haben sich in der Praxis bewährt.

Erfindungsgemäß ist, daß das Komposit aus einer fließfähigen Suspension, die aus einer homogenen Mischung von Propan-2-on und/oder Butan-2-on, einem oder mehreren hydrophoben Polymeren aus den Gruppen der Poly(methacrylsäureester), der Poly(acrylsäureester) und der Poly(methacrylsäureester-co-acrylsäureester) und einem oder mehreren hydrophilen Polymeren aus den Gruppen der Polyether besteht, in der ein oder mehrere in Wasser gering lösliche Antibiotika aus den Gruppen der Aminoglykosid-Antibiotika, der Lincosamid-Antibiotika, der Tetracyclin-Antibiotika und der Chinolon-Antibiotika, einem in Wasser leichtlöslichem Antibiotikum aus den Gruppen der Aminoglykosid-Antibiotika, der Lincosamid-Antibiotika, der β-Lactam-Antibiotika und der Tetracyclin-Antibiotika, und ggf. ein oder mehrere organische Hilfsstoffe, suspendiert sind, durch Verdampfung von Propan-2-on und/oder Butan-2-on, gebildet wird.

Ebenfalls erfindungsgemäß ist, daß das Komposit aus einer Schmelze gebildet wird, die aus einem oder mehreren hydrophoben Polymeren aus den Gruppen der Poly(methacrylsäureester), der Poly(acrylsäureester), der Poly(methacrylsäureester-co-acrylsäureester) und einem oder mehreren hydrophilen Polymeren aus den Gruppen der Polyether besteht, in der ein oder mehrere in Wasser gering lösliche Antibiotika aus den Gruppen der Aminoglykosid-Antibiotika, der Lincosamid-Antibiotika, der Tetracyclin-Antibiotika und der Chinolon-Antibiotika, einem in Wasser leichtlöslichem Antibiotikum aus den Gruppen der Aminoglykosid-Antibiotika, der Lincosamid-Antibiotika und der Tetracyclin-Antibiotika, und ggf. ein oder mehrere organische Hilfsstoffe, suspendiert sind.

Weiterhin ist erfindungsgemäß, daß der Gehalt an hydrophilem Polymer in der homogenen Polymermischung zwischen 0,1 bis 60 Masseprozent beträgt.

Erfindungsgemäß ist, daß Polyethylenglykol mit einer mittleren Molmasse im Bereich von 120 gmol⁻¹ bis 35000 gmol⁻¹ als Polyether bevorzugt wird.

Ebenfalls erfindungsgemäß ist, daß Polypropylenglykol mit einer mittleren Molmasse im Bereich von 200 gmol⁻¹ bis 35000 gmol⁻¹ als Polyether bevorzugt wird.

Erfindungsgemäß ist, daß Polyethylenglykol mit einer mittleren Molmasse im Bereich von 200 gmol⁻¹ bis 600 gmol⁻¹ als Polyether besonders bevorzugt wird.

Erfindungsgemäß ist, daß Poly(methacrylsäuremethylester), Poly(methacrylsäureethylester), Poly(methacrylsäurepropylester), Poly(methacrylsäure-n-butylester), Poly(methacrylsäure-nhexylester),Poly(methacrylsäurecyclohexylester), Poly(acrylsäuremethylester), Poly(acrylsäureethylester), Poly(acrylsäurepropylester), Poly(acrylsäurebutylester) und Poly(acrylsäurecyclohexylester) mit mittleren Molmassen von 20 000 gmol⁻¹ bis 1 000 000 gmol⁻¹ als hydrophobe Polymere bevorzugt sind.

Auch erfindungsgemäß ist, daß Copolymere und Terpolymere mit mittleren Molmassen von 20 000 gmol⁻¹ bis 1 000 000 gmol⁻¹ als hydrophobe Polymere bevorzugt sind, die aus Acrylsäuremethylester, Acrylsäureethylester, Acrylsäurepropylester, Acrylsäure-n-hexylester, Acrylsäurecyclohexylester, Methacrylsäuremethylester, Methacrylsäureethylester, Methacrylsäurepropylester, Methacrylsäurebutylester, Methacrylsäure-n-hexylester und Methacrylsäurecyclohexylester hergestellt sind.

Erfindungsgemäß ist, daß Sulfonamide und/oder Antiphlogistika und/oder Anästhetika und/oder Vancomycin als organische Hilfsstoffe bevorzugt werden.

Erfindungsgemäß ist, daß die fließfähige Suspension durch Spinnen, unter Verdampfung von Propan-2-on und/oder Butan-2-on, Komposite in Form von Fäden bildet.

Erfindungsgemäß ist, daß die fließfähige Suspension durch Gießen, unter Verdampfung von Propan-2-on und/oder Butan-2-on, Komposite in Form von Folien bildet.

Erfindungsgemäß ist, daß die fließfähige Suspension durch Versprühen, unter Verdampfung von Propan-2-on und/oder Butan-2-on, Komposite in Form von Pulvern und Granulaten bildet.

Erfindungsgemäß ist, daß das Komposit durch Pressen, Strangpressen und Walzen zu Formkörpern und Folien geformt wird.

Erfindungsgemäß ist, daß die mit dem Komposit beschichteten Kunststoffschläuche, Kunststoffäden, Kunststoffolien, kugelförmigen Kunststoffkörper, walzenförmigen Kunststoffkörper und kettenförmigen Kunststoffkörper als medizinische Implantate verwendet werden.

Erfindungsgemäß ist, daß Katheter, Tracheal-Kanülen und Schläuche zur intraperitonealen Ernährung mit dem Komposit beschichtet sind.

Erfindungsgemäß ist, daß implantierbare Metallplatten, Metallnägel, Metallschrauben mit dem Komposit beschichtet sind.

Weiterhin ist im Sinne der Erfindung, daß das Komposit zur Verklebung von medizinisch verwendbaren Kunststoffformkörpern, Kunststoffolien, Kunststoffäden, Metallplatten und Metallröhren verwendet wird.

Erfindungsgemäß ist, daß das Komposit als Bindemittel zur Herstellung von antibiotischen Formkörpern aus Kunststoffgranulaten, Kunststoffpulvern, resorbierbaren Glaspulvern, nichtresorbierbaren Glaspulvern und Quarz-Pulvern verwendet wird.

Erfindungsgemäß ist, daß die fließfähige Suspension durch Tauchen, Sprühen, Streichen, Bürsten und Walzen auf die Oberfläche von Kunststoffen und/oder Metallen aufgebracht wird und durch Verdampfung von Propan-2-on und/oder Butan-2-on ein Komposit in Form einer Beschichtung bildet.

Erfindungsgemäß ist, daß das Komposit als Beschichtung auf medizinisch verwendbaren Kunststoffäden, Kunststoffolien, Kunststoffschläuche, Kunststoffbeutel und Kunststofflaschen aufgebracht wird.

Erfindungsgemäß ist, daß der Komposit als Beschichtung auf kugelförmige Formkörper, auf walzenförmige Formkörper und auf kettenförmige Formkörper aufgebracht wird, die aus Kunststoff und/oder Metall bestehen.

Ferner ist erfindungsgemäß, daß das Komposit als Beschichtung auf Formkörper, Folien und Fäden aus Poly(methacrylsäureester), Poly(acrylsäureester), Poly(methacrylsäureester-coacrylsäureester), Polyvinylchlorid, Polyvinylidenchlorid, Silikon, Polystyren und Polycarbonat aufgebracht wird.

Erfindungsgemäß ist ebenfalls, daß der Komposit als Bindemittel zur Herstellung von antibiotischen Laminaten verwendet wird.

Weiterhin ist erfindungsgemäß, daß das Komposit durch Sintern als Beschichtung auf die Oberfläche von Metallen und/oder Kunststoffen aufgebracht wird.

Die Erfindung soll an drei Beispielen näher erläutert werden.

### Beispiel 1:

Es wird eine Lösung bestehend aus 1,5g Poly(methylmethacrylat), 120 mg Polyethylenglykol 600 und 5 ml Aceton hergestellt. In diese Lösung werden 300 mg feingepulvertes Gentamicinpentakis-hexadecylsulfonat und 300 mg Gentamicinsulfat suspendiert. Diese Suspension wird auf eine Glasplatte gegossen. Man läßt das Aceton eindampfen. Dabei entsteht eine halbtransparente, elastische Folie, die sich von der Glasplatte abziehen läßt.

### Beispiel 2:

Es wird eine Lösung bestehend aus 1,5g Poly(methylmethacrylat), 120 mg Polyethylenglykol 600 und 5 ml Aceton hergestellt. In diese Lösung werden 300 mg feingepulvertes Gentamicin-pentakis-dodecylsulfat und 300 mg Gentamicinsulfat suspendiert. In diese Suspension taucht man ein 3 cm langes Stück Polyvinylchloridschlauch (Schlauch-Durchmesser 4 mm). Anschließend läßt man den beschichteten Polyvinylchloridschlauch bei Raumtemperatur trocknen. Man erhält eine elastische haftfeste Beschichtung auf dem Polyvinylchloridschlauch.

### Vergleichs-Beispiel 3:

In eine Schmelze (150°C) von 2g Poly(methacrylsäure-co-acrylsäuremethylester und 200mg Polyethylenglykol 600 werden 200mg feingepulvertes Gentamicin-pentakis-dodecylsulfat eingebracht und gleichmäßig verteilt wird. Nach Erkalten der Schmelze erhält man ein milchig-trübes, festes Komposit.

## Patentansprüche

1. Antibiotikum-/Antibiotika-Polymer-Kombination, **dadurch gekennzeichnet, dass** in einer homogenen Polymermischung, die aus einem oder mehreren hydrophoben Polymeren aus den Gruppen der Poly(methacrylsäureester), der Poly(acrylsäureester) und der Poly(methacrylsäureester-co-acrylsäureester) und aus einem oder mehreren hydrophilen Polymeren aus den Gruppen der Polyether besteht, ein oder mehrere in Wasser gering lösliche Antibiotika aus den Gruppen der Aminoglykosid-Antibiotika, der Lincosamid-Antibiotika, der Tetracyclin-Antibiotika, der Chinolon-Antibiotika, ein in Wasser leichtlösliches Antibiotikum aus den Gruppen der Aminoglykosid-Antibiotika, der Lincosamid-Antibiotika, der β-Lactam-Antibiotika und der Tetracyclin-Antibiotika, und ggf. ein oder mehrere organische Hilfsstoffe, suspendiert sind und diese Suspension ein Komposit bildet.

2. Antibiotikum-/Antibiotika-Polymer-Kombination nach Anspruch 1, **dadurch gekennzeichnet, dass** das Komposit aus einer fließfähigen Suspension, die aus einer homogenen Mischung von Propan-2-on und/oder Butan-2-on, einem oder mehreren hydrophoben Polymeren aus den Gruppen der Poly(methacrylsäureester), der Poly(acrylsäureester) und der Poly(methacrylsäureester-co-acrylsäureester) und einem oder mehreren hydrophilen Polymeren aus den Gruppen der Polyether besteht, in der ein oder mehrere in Wasser gering lösliche Antibiotika aus den Gruppen der Aminoglykosid-Antibiotika, der Lincosamid-Antibiotika, der Tetracyclin-Antibiotika und der Chinolon-Antibiotika, ein in Wasser leichtlösliches Antibiotikum aus den Gruppen der Aminoglykosid-Antibiotika, der Lincosamid-Antibiotika, der β-Lactam-Antibiotika und der Tetracyclin-Antibiotika, und ggf. ein oder mehrere organische Hilfsstoffe, suspendiert sind, durch Verdampfung von Propan-2-on und/oder Butan-2-on, gebildet wird.

3. Antibiotikum-/Antibiotika-Polymer-Kombination nach Anspruch 1, **dadurch gekennzeichnet, dass** das Komposit aus einer Schmelze gebildet wird, die aus einem oder mehreren hydrophoben Polymeren aus den Gruppen der Poly(methacrylsäureester), der Poly(acrylsäureester), der Poly(methacrylsäureester-co-acrylsäureester) und einem oder mehreren hydrophilen Polymeren aus den Gruppen der Polyether besteht, in der ein oder mehrere in Wasser gering lösliche Antibiotika aus den Gruppen der Aminoglykosid-Antibiotika, der Lincosamid-Antibiotika, der Tetracyclin-Antibiotika und der Chinolon-Antibiotika, ein in Wasser leichtlösliches Antibiotikum aus den Gruppen der Aminoglykosid-Antibiotika, der Lincosamid-Antibiotika und der Tetracyclin-Antibiotika, und ggf. ein oder mehrere organische Hilfsstoffe, suspendiert sind.

4. Antibiotikum-/Antibiotika-Polymer-Kombination nach einem der Ansprüche 1 bis 3, **dadurch gekennzeichnet, dass** der Gehalt an hydrophilem Polymer in der homogenen Polymermischung zwischen 0,1 bis 60 Masseprozent beträgt.

5. Antibiotikum-/Antibiotika-Polymer-Kombination nach einem der Ansprüche 1 bis 4, **dadurch gekennzeichnet, dass** Polyethylenglykol mit einer mittleren Molmasse im Bereich von 120 gmol⁻¹ bis 35.000 gmol⁻¹ als Polyether verwendet wird.

6. Antibiotikum-/Antibiotika-Polymer-Kombination nach einem der Ansprüche 1 bis 3, **dadurch gekennzeichnet, dass** Polypropylenglykol mit einer mittleren Molmasse im Bereich von 200 gmol⁻¹ bis 35.000 gmol⁻¹ als Polyether verwendet wird.

7. Antibiotikum-/Antibiotika-Polymer-Kombination nach einem der Ansprüche 1 bis 5, **dadurch gekennzeichnet, dass** Polyethylenglykol mit einer mittleren Molmasse im Bereich von 120 gmol⁻¹ bis 600 gmol⁻¹ als Polyether verwendet wird.

8. Antibiotikum-/Antibiotika-Polymer-Kombination nach einem der Ansprüche 1 bis 7, **dadurch gekennzeichnet, dass** Poly(methacrylsäuremethylester), Poly(methacrylsäureethylester), Poly(methacryl-säurepropylester), Poly(methacrylsäure-n-butylester), Poly(methacrylsäuren-hexyl-ester),Poly(methacrylsäurecyclohexylester), Poly(acrylsäuremethylester), Poly(acrylsäureethylester), Poly(acrylsäurepropylester), Poly(acrylsäurebutylester) und Poly(acryl-säurecyclohexylester) mit mittleren Molmassen von 20.000 gmol⁻¹ bis 1.000.000 gmol⁻¹ als hydrophobe Polymere verwendet wird.

9. Antibiotikum-/Antibiotika-Polymer-Kombination nach einem der Ansprüche 1 bis 7, **dadurch gekennzeichnet, dass** Copolymere und Terpolymere mit mittleren Molmassen von 20.000 gmol⁻¹ bis 1.000.000 gmol⁻¹als hydrophobe Polymere verwendet werden, die aus Acrylsäuremethylester, Acrylsäureethylester, Acrylsäurepropylester, Acrylsäure-n-hexylester, Acrylsäure-cyclohexylester, Methacrylsäuremethylester, Methacrylsäureethylester, Methacrylsäurepropylester, Methacrylsäurebutylester, Methacrylsäure-n-hexylester und Methacrylsäurecyclohexylester hergestellt sind.

10. Antibiotikum-/Antibiotika-Polymer-Kombination nach einem der Ansprüche 1 bis 7, **dadurch gekennzeichnet, dass** Sulfonamide und/oder Antiphlogistika und/oder Anästhetika als organische Hilfsstoffe verwendet werden.

11. Antibiotikum-/Antibiotika-Polymer-Kombination nach einem der Ansprüche 2 oder 4 bis 10, **dadurch gekennzeichnet, dass** die fließfähige Suspension durch Spinnen, unter Verdampfung von Propan-2-on und/oder Butan-2-on, Komposite in Form von Fäden bildet.

12. Antibiotikum-/Antibiotika-Polymer-Kombination nach einem der Ansprüche 2 oder 4 bis 10, **dadurch gekennzeichnet, dass** die fließfähige Suspension durch Gießen, unter Verdampfung von Propan-2-on und/oder Butan-2-on, Komposite in Form von Folien bildet.

13. Antibiotikum-/Antibiotika-Polymer-Kombination nach einem der Ansprüche 2 oder 4 bis 10, **dadurch gekennzeichnet, dass** die fließfähige Suspension durch Versprühen, unter Verdampfung von Propan-2-on und/oder Butan-2-on, Komposite in Form von Pulvern und Granulaten bildet.

14. Antibiotikum-/Antibiotika-Polymer-Kombination nach einem der Ansprüche 1 bis 13, **dadurch gekennzeichnet, dass** das Komposit durch Pressen, Strangpressen und Walzen zu Formkörpern und Folien geformt wird.

15. Antibiotikum-/Antibiotika-Polymer-Kombination nach einem der Ansprüche 1 bis 14, **dadurch gekennzeichnet, dass** die mit dem Komposit beschichteten Kunstoffschläuche, Kunstofffäden, Kunstofffolien, kugelförmigen Kunstoffkörper, walzenförmigen Kunstoffkörper und kettenförmigen Kunsstoffkörper als medizinische Implantate verwendet werden.

16. Antibiotikum-/Antibiotika-Polymer-Kombination nach einem der Ansprüche 1 bis 14, **dadurch gekennzeichnet, dass** Katheter, Tracheal-Kanülen und Schläuche zur intraperitonealen Ernährung mit dem Komposit beschichtet sind.

17. Antibiotikum-/Antibiotika-Polymer-Kombination nach einem der Ansprüche 1 bis 14, dadurch gekenneichnet, dass implantierbare Metallplatten, Metallnägel, Metallschrauben mit dem Komposit beschichtet sind.

18. Antibiotikum-/Antibiotika-Polymer-Kombination nach einem der Ansprüche 1 bis10, **dadurch gekennzeichnet, dass** das Komposit zur Verklebung von medizinisch verwendbaren Kunstofformkörpern, Kunststoffolien, Kunststoffäden, Metallplatten und Metallröhren verwendet wird.

19. Antibiotikum-/Antibiotika-Polymer-Kombination nach einem der Ansprüche 1 bis10, **dadurch gekennzeichnet, dass** das Komposit als Bindemittel zur Herstellung von antibiotischen Formkörpern aus Kunststoffgranulaten, Kunststoffpulvern, resorbierbaren Glaspulvern, nichtresorbierbaren Glaspulvern und Quarz-Pulvern verwendet wird.

20. Antibiotikum-/Antibiotika-Polymer-Kombination nach einem der Ansprüchen 1 bis 10, **dadurch gekennzeichnet, dass** das Komposit als Bindemittel zur Herstellung von antibiotischen Laminaten verwendet wird.

21. Verwendung einer Antibiotikum-/Antibiotika-Polymer-Kombination nach einem der Ansprüche 2 oder 4 bis 10, **dadurch gekennzeichnet, dass** die fließfähige Suspension durch Tauchen, Sprühen, Streichen, Bürsten und Walzen auf die Oberfläche von Kunststoffen und/oder Metallen aufgebracht wird und durch Verdampfung von Propan-2-on und/oder Butan-2-on einen Komposit in Form einer Beschichtung bildet.

22. Verwendung einer Antibiotikum-/Antibiotika-Polymer-Kombination nach einem der Ansprüche 1 bis 10, **dadurch gekennzeichnet, dass** das Komposit als Beschichtung auf medizinisch verwendbaren Kunststoffäden, Kunststoffolien, Kunststoffschläuche, Kunststoffbeutel und Kunststofflaschen aufgebracht wird.

23. Verwendung einer Antibiotikum-/Antibiotika-Polymer-Kombination nach einem der Ansprüche 1 bis 10, **dadurch gekennzeichnet, dass** das Komposit als Beschichtung auf kugelförmige Formkörper, auf walzenförmige Formkörper und auf kettenförmige Formkörper aufgebracht wird, die aus Kunststoff und/oder Metall bestehen.

24. Verwendung einer Antibiotikum-/Antibiotika-Polymer-Kombination nach einem der Ansprüche 1 bis 10, **dadurch gekennzeichnet, dass** das Komposit als Beschichtung auf Formkörper, Folien und Fäden aus Poly(methacrylsäureester), Poly(acrylsäureester), Poly(methacryl-säureester-co-acrylsäureester), Polyvinylchlorid, Polyvinylidenchlorid, Silikon, Polystyren und Polycarbonat aufgebracht wird.

25. Verwendung einer Antibiotikum-/Antibiotika-Polymer-Kombination nach einem der Ansprüche 1 oder 3, **dadurch gekennzeichnet, dass** das Komposit durch Sintern als Beschichtung auf die Oberfläche von Metallen und/oder Kunststoffen aufgebracht wird.

## Claims

1. Antibiotic/antibiotics-polymer combination, **characterized in that** one or more sparingly water-soluble antibiotics from the groups consisting of the aminoglycoside antibiotics, the lincosamide antibiotics, the tetracycline antibiotics and the quinoline antibiotics, a freely-soluble antibiotic from the groups consisting of the aminoglycoside antibiotics, lincosamide antibiotics, the β-lactam antibiotics and the tetracycline antibiotics, and optionally one or more organic auxiliaries, are suspended in a homogeneous polymer blend which consists of one or more hydrophobic polymers from the groups consisting of the poly(methacrylic esters), the poly(acrylic esters) and the poly(methacrylic ester-co-acrylic esters) and of one or more hydrophilic polymers from the groups consisting of the polyethers, and this suspension forms a composite.

2. Antibiotic/antibiotics-polymer combination according to Claim 1, **characterized in that** the composite is formed from a flowable suspension which consists of a homogeneous mixture of propan-2-one and/or butan-2-one, one or more hydrophobic polymers from the groups consisting of the poly(methacrylic esters), the poly(acrylic esters) and the poly(methacrylic ester-co-acrylic esters) and one or more hydrophilic polymers from the groups consisting of the polyethers, in which suspension one or more sparingly water-soluble antibiotics from the groups consisting of the aminoglycoside antibiotics, the lincosamide antibiotics, the tetracycline antibiotics and the quinoline antibiotics, a freely water-soluble antibiotic from the groups consisting of the aminoglycoside antibiotics, the lincosamide antibiotics, the β-lactam antibiotics and the tetracycline antibiotics, and optionally one or more organic auxiliaries, are suspended, by evaporation of propan-2-one and/or butan-2-one.

3. Antibiotic/antibiotics-polymer combination according to Claim 1, **characterized in that** the composite is formed from a melt which consists of one or more hydrophobic polymers from the groups consisting of the poly(methacrylic esters), the poly(acrylic esters), the poly(methacrylic ester-co-acrylic esters) and one or more hydrophilic polymers from the groups consisting of the polyethers, in which melt one or more sparingly water-soluble antibiotics from the group consisting of the aminoglycoside antibiotics, the lincosamide antibiotics, the tetracycline antibiotics and the quinoline antibiotics, a freely water-soluble antibiotic from the groups consisting of the aminoglycoside antibiotics, the lincosamide antibiotics and the tetracycline antibiotics, and optionally one or more organic auxiliaries, are suspended.

4. Antibiotic/antibiotics-polymer combination according to any of Claims 1 to 3, **characterized in that** the content of hydrophilic polymer in the homogeneous polymer blend is between 0.1 and 60 percent by mass.

5. Antibiotic/antibiotics-polymer combination according to any of Claims 1 to 4, **characterized in that** polyethylene glycol having an average molar mass in the range from 120 gmol⁻¹ to 35,000 gmol⁻¹ is used as the polyether.

6. Antibiotic/antibiotics-polymer combination according to any of Claims 1 to 3, **characterized in that** polypropylene glycol having an average molar mass in the range from 200 gmol⁻¹, to 35,000 gmol⁻¹ is used as the polyether.

7. Antibiotic/antibiotics-polymer combination according to any of Claims 1 to 5, **characterized in that** polyethylene glycol having an average molar mass in the range from 120 gmol⁻¹ to 600 gmol⁻¹ is used as the polyether.

8. Antibiotic/antibiotics-polymer combination according to any of Claims 1 to 7, **characterized in that** poly(methyl methacrylate), poly(ethyl methacrylate), poly(propyl methacrylate), poly(n-butyl methacrylate), poly(n-hexyl methacrylate), poly(cyclohexyl methacrylate), poly(methyl acrylate), poly(ethyl acrylate), poly(propyl acrylate), poly(butyl acrylate) and poly(cyclohexyl acrylate) having average molar masses of from 20,000 gmol⁻¹ to 1,000,000 gmol⁻¹ are used as hydrophobic polymers.

9. Antibiotic/antibiotics-polymer combination according to any of Claims 1 to 7, **characterized in that** copolymers and terpolymers having average molar masses of from 20,000 gmol⁻¹ to 1,000,000 gmol⁻¹ are used as hydrophobic polymers, which are prepared from methyl acrylate, ethyl acrylate, propyl acrylate, n-hexyl acrylate, cyclohexyl acrylate, methyl methacrylate, ethyl methacrylate, propyl methacrylate, butyl methacrylate, n-hexyl methacrylate and cyclohexyl methacrylate.

10. Antibiotic/antibiotics-polymer combination according to any of Claims 1 to 7, **characterized in that** sulphonamides and/or antiphlogistic agents and/or anaesthetics are used as organic auxiliaries.

11. Antibiotic/antibiotics-polymer combination according to any of Claims 2 or 4 to 10, **characterized in that** the flowable suspension forms composites in the form of filaments by spinning, with evaporation of propan-2-one and/or butan-2-one.

12. Antibiotic/antibiotics-polymer combination according to any of Claims 2 or 4 to 10, **characterized in that** the flowable suspension forms composites in the form of films by casting, with evaporation of propan-2-one and/or butan-2-one.

13. Antibiotic/antibiotics-polymer combination according to any of Claims 2 or 4 to 10, **characterized in that** the flowable suspension forms composites in the form of powders and granules by atomization, with evaporation of propan-2-one and/or butan-2-one.

14. Antibiotic/antibiotics-polymer combination according to any of Claims 1 to 13, **characterized in that** the composite is shaped by pressing, extrusion and rolling to give mouldings and films.

15. Antibiotic/antibiotics-polymer combination according to any of Claims 1 to 14, **characterized in that** the plastic tubes, plastic filaments, plastic films, spherical plastic bodies, roll-like plastic bodies and chain-like plastic bodies coated with the composite are used as medical implants.

16. Antibiotic/antibiotics-polymer combination according to any of Claims 1 to 14, **characterized in that** catheters, tracheal cannulae and tubes for intraperitoneal feeding are coated with the composite.

17. Antibiotic/antibiotics-polymer combination according to any of Claims 1 to 14, **characterized in that** implantable metal plates, metal pins, or metal screws are coated with the composite.

18. Antibiotic/antibiotics-polymer combination according to any of Claims 1 to 10, **characterized in that** the composite is used for the adhesive bonding of plastic mouldings, plastic films, plastic filaments, metal plates and metal tubes which can be used in medicine.

19. Antibiotic/antibiotics-polymer combination according to any of Claims 1 to 10, **characterized in that** the composite is used as a binder for the production of antibiotic mouldings from plastic granules, plastic powders, absorbable glass powders, nonabsorbable glass powders and quartz powders.

20. Antibiotic/antibiotics-polymer combination according to any of Claims 1 to 10, **characterized in that** the composite is used as a binder for the production of antibiotic laminates.

21. Use of an antibiotic/antibiotics-polymer combination according to any of Claims 2 or 4 to 10, **characterized in that** the flowable suspension is applied to the surface of plastics and/or metals by immersion, spraying, spreading, brushing and rolling, and a composite in the form of a coating is formed by evaporation of propan-2-one and/or butan-2-one.

22. Use of an antibiotic/antibiotics-polymer combination according to any of Claims 1 to 10, **characterized in that** the composite is applied as a coating to plastic filaments, plastic films, plastic tubes, plastic bags and plastic bottles which can be used in medicine.

23. Use of an antibiotic/antibiotics-polymer combination according to any of Claims 1 to 10, **characterized in that** the composite is applied as a coating to spherical mouldings, to roll-like mouldings and to chain-like mouldings which consist of plastic and/or metal.

24. Use of an antibiotic/antibiotics-polymer combination according to any of Claims 1 to 10, **characterized in that** the composite is applied as a coating to mouldings, films and filaments of poly(methacrylic esters), poly(acrylic esters), poly(methacrylic ester-co-acrylic esters), polyvinyl chloride, polyvinylidene chloride, silicone, polystyrene and polycarbonate.

25. Use of an antibiotic/antibiotics-polymer combination according to either of Claims 1 and 3, **characterized in that** the composite is applied as a coating to the surface of metals and/or plastics by sintering.

## Revendications

1. Combinaison antibiotique/antibiotiques-polymères **caractérisée en ce qu'**un ou plusieurs antibiotiques peu solubles dans l'eau des groupes des antibiotiques aminoglycosides, des antibiotiques lincosamides, des antibiotiques tétracyclines, des antibiotiques quinolones, un antibiotique très soluble dans l'eau des groupes des antibiotiques aminoglycosides, des antibiotiques lincosamides, des antibiotiques β-lactames et des antibiotiques tétracyclines, et éventuellement un ou plusieurs adjuvants organiques, sont mis en suspension dans un mélange de polymères homogène qui consiste en un ou plusieurs polymères hydrophobes des groupes des poly(esters de l'acide méthacrylique), des poly(esters de l'acide acrylique) et des poly(esters de l'acide méthacrylique-co-esters de l'acide acrylique) et en un ou plusieurs polymères hydrophiles des groupes des polyéthers, et cette suspension forme un composite.

2. Combinaison antibiotique/antibiotiques-polymères selon la revendication 1 **caractérisée en ce que** le composite est formé à partir d'une suspension fluide, qui consiste en un mélange homogène de propan-2-one et/ou de butan-2-one, un ou plusieurs polymères hydrophobes des groupes des poly(esters de l'acide méthacrylique), des poly(esbers de l'acide acrylique) et des poly(esters de l'acide méthacrylique-co-esters de l'acide acrylique) et un ou plusieurs polymères hydrophiles des groupes des polyéthers, dans laquelle un ou plusieurs antibiotiques peu solubles dans l'eau des groupes des antibiotiques aminoglycosides, des antibiotiques lincosamides, des antibiotiques tétracyclines et des antibiotiques quinolones, un antibiotique très soluble dans l'eau des groupes des antibiotiques aminoglycosides, des antibiotiques lincosamides, des antibiotiques β-lactames et des antibiotiques tétracyclines, et éventuellement un ou plusieurs adjuvants organiques, sont mis en suspension, par évaporation de la propan-2-one et/ou de la butan-2-one.

3. Combinaison antibiotique/antibiotiques-polymères selon la revendication 1 **caractérisée en ce que** le composite est formé à partir d'une masse fondue qui consiste en un ou plusieurs polymères hydrophobes des groupes des poly(esters de l'acide méthacrylique), des poly(esters de l'acide acrylique), des poly(esters de l'acide méthacrylique-co-esters de l'acide acrylique) et un ou plusieurs polymères hydrophiles des groupes des polyéthers, dans laquelle un ou plusieurs antibiotiques peu solubles dans l'eau des groupes des antibiotiques aminoglycosides, des antibiotiques lincosamides, des antibiotiques tétracyclines et des antibiotiques quinolones, un antibiotique très soluble dans l'eau des groupes des antibiotiques aminoglycosides, des antibiotiques lincosamides et des antibiotiques tétracyclines, et éventuellement un ou plusieurs adjuvants organiques, sont mis en suspension.

4. Combinaison antibiotique/antibiotiques-polymères selon l'une des revendications 1 à 3 **caractérisée en ce que** la teneur en polymère hydrophile dans le mélange de polymères homogène est entre 0,1 et 60 % en masse.

5. Combinaison antibiotique/antibiotiques-polymères selon l'une des revendications 1 à 4 **caractérisée en ce qu'**un polyéthylèneglycol ayant une masse molaire moyenne dans le domaine de 120 gmol⁻¹ à 35.000 gmol⁻¹ est utilisé comme polyéther.

6. Combinaison antibiotique/antibiotiques-polymères selon l'une des revendications 1 à 3 **caractérisée en ce qu'**un polypropylèneglycol ayant une masse molaire moyenne dans le domaine de 200 gmol⁻¹ à 35.000 gmol⁻¹ est utilisé comme polyéther.

7. Combinaison antibiotique/antibiotiques-polymères selon l'une des revendications 1 à 5 **caractérisée en ce qu'**un polyéthylèneglycol ayant une masse molaire moyenne dans le domaine de 120 gmol⁻¹ à 600 gmol⁻¹ est utilisé comme polyéther.

8. Combinaison antibiotique/antibiotiques-polymères selon l'une des revendications 1 à 7 **caractérisée en ce qu'**un poly(méthacrylate de méthyle), un poly(méthacrylate d'éthyle), un poly(méthacrylate de propyle), un poly(méthacrylate de n-butyle), un poly(méthacrylate de n-hexyle), un poly(méthacrylate de cyclohexyle), un poly(acrylate de méthyle), un poly(acrylate d'éthyle), un poly(acrylate de propyle), un poly(acrylate de butyle) et un poly(acrylate de cyclohexyle) ayant des masses molaires moyennes de 20000 gmol⁻¹ à 1000000 gmol⁻¹ est utilisé comme polymères hydrophobes.

9. Combinaison antibiotique/antibiotiques-polymères selon l'une des revendications 1 à 7 **caractérisée en ce que** des copolymères et des terpolymères ayant des masses molaires moyennes de 20.000 gmol⁻¹ à 1.000.000 gmol⁻¹, qui sont préparés à partir d'acrylate de méthyle, d'acrylate d'éthyle, d'acrylate de propyle, d'acrylate de n-hexyle, d'acrylate de cydohexyle, de méthacrylate de méthyle, de méthacrylate d'éthyle, de méthacrylate de propyle, de méthacrylate de butyle, de méthacrylate de n-hexyle et de méthacrylate de cydohexyle, sont utilisés comme polymères hydrophobes.

10. Combinaison antibiotique/antibiotiques-polymères selon l'une des revendications 1 à 7 **caractérisée en ce que** des sulfonamides et/ou des antiphlogistiques et/ou des anesthésiques sont utilisés comme adjuvants organiques.

11. Combinaison antibiotique/antibiotiques-polymères selon l'une des revendications 2 ou 4 à 10 **caractérisée en ce que** la suspension fluide forme des composites sous forme de fils par filage, avec évaporation de propan-2-one et/ou de butan-2-one.

12. Combinaison antibiotique/antibiotiques-polymères selon l'une des revendications 2 ou 4 à 10 **caractérisée en ce que** la suspension fluide forme des composites sous forme de feuilles par coulée, avec évaporation de propan-2-one et/ou de butan-2-one.

13. Combinaison antibiotique/antibiotiques-polymères selon l'une des revendications 2 ou 4 à 10 **caractérisée en ce que** la suspension fluide forme des composites sous forme de poudres et de granulés par pulvérisation, avec évaporation de propan-2-one et/ou de butan-2-one.

14. Combinaison antibiotique/antibiotiques-polymères selon l'une des revendications 1 à 13 **caractérisée en ce que** le composite est mis sous forme de corps mis en forme et de feuilles par compression, extrusion et laminage.

15. Combinaison antibiotique/antibiotiques-polymères selon l'une des revendications 1 à 14 **caractérisée en ce que** les tuyaux de matière plastique, les fils de matière plastique, les feuilles de matière plastique, les corps de matière plastique sphériques, les corps de matière plastique cylindriques et les corps de matière plastiques en forme de chaîne revêtus du composite sont utilisés comme implants médicaux.

16. Combinaison antibiotique/antibiotiques-polymères selon l'une des revendications 1 à 14 **caractérisée en ce que** des cathéters, des canules trachéales et des tuyaux pour l'alimentation intrapéritonéale sont revêtus du composite.

17. Combinaison antibiotique/antibiotiques-polymères selon l'une des revendications 1 à 14 **caractérisée en ce que** des plaques métalliques, des broches métalliques, des vis métalliques implantables sont revêtues du composite.

18. Combinaison antibiotique/antibiotiques-polymères selon l'une des revendications 1 à 10 **caractérisée en ce que** le composite est utilisé pour le collage de corps mis en forme en matière plastique, de feuilles de matière plastique, de fils de matière plastique, de plaques métalliques et de tubes métalliques utilisables du point de vue médical.

19. Combinaison antibiotique/antibiotiques-polymères selon l'une des revendications 1 à 10 **caractérisée en ce que** le composite est utilisé comme liant pour la production de corps mis en forme antibiotiques à partir de granulés de matière plastique, de poudres de matière plastique, de poudres de verre résorbables, de poudres de verre non résorbables et de poudres de quartz.

20. Combinaison antibiotique/antibiotiques-polymères selon l'une des revendications 1 à 10 **caractérisée en ce que** le composite est utilisé comme liant pour la production de stratifiés antibiotiques.

21. Utilisation d'une combinaison antibiotique/antibiotiques-polymères selon l'une des revendications 2 ou 4 à 10 **caractérisée en ce que** la suspension fluide est appliquée par immersion, pulvérisation, enduction, à la brosse et au rouleau sur la surface de matières plastiques et/ou de métaux et forme un composite sous forme d'un revêtement par évaporation de propan-2-one et/ou de butan-2-one.

22. Utilisation d'une combinaison antibiotique/antibiotiques-polymères selon l'une des revendications 1 à 10 **caractérisée en ce que** le composite est appliqué sous forme de revêtement sur des fils de matière plastique, des feuilles de matière plastique, des tuyaux de matière plastique, des sachets de matière plastique et des bouteilles de matière plastique utilisables du point de vue médical.

23. Utilisation d'une combinaison antibiotique/antibiotiques-polymères selon l'une des revendications 1 à 10 **caractérisée en ce que** le composite est appliqué sous forme de revêtement sur des corps mis en forme sphériques, sur des corps mis en forme cylindriques et sur des corps mis en forme en forme de chaîne qui consistent en matière plastique et/ou en métal.

24. Utilisation d'une combinaison antibiotique/antibiotiques-polymères selon l'une des revendications 1 à 10 **caractérisée en ce que** le composite est appliqué sous forme de revêtement sur des corps mis en forme, des feuilles et des fils en poly(esters de l'acide méthacrylique), en poly(esters de l'acide acrylique), en poly(esters de l'acide méthacrylique-co-esters de l'acide acrylique), en poly(chlorure de vinyle), en poly(chlorure de vinylidène), en silicone, en polystyrène et en polycarbonate.

25. Utilisation d'une combinaison antibiotique/antibiotiques-polymères selon l'une des revendications 1 ou 3 **caractérisée en ce que** le composite est appliqué par frittage sous forme de revêtement sur la surface de métaux et/ou de matières plastiques.
